(19) Europäisches Patentamt
European Patent Office
Office européen des brevets

(11) Veröffentlichungsnummer: **0 060 429**
**B1**

(12) # EUROPÄISCHE PATENTSCHRIFT

(45) Veröffentlichungstag der Patentschrift: **25.01.84**

(51) Int. Cl.³: **C 07 D 215/18**, C 07 D 215/48, C 07 D 401/12, A 01 N 43/42

(21) Anmeldenummer: **82101534.4**

(22) Anmeldetag: **27.02.82**

(54) **Dichlorchinolinderivate, ihre Herstellung, ihre Verwendung als Herbizide und Mittel dafür.**

(30) Priorität: **09.03.81 DE 3108873**

(43) Veröffentlichungstag der Anmeldung: **22.09.82 Patentblatt 82/38**

(45) Bekanntmachung des Hinweises auf die Patenterteilung: **25.01.84 Patentblatt 84/4**

(84) Benannte Vertragsstaaten: **AT BE CH DE FR GB IT LI LU NL SE**

(56) Entgegenhaltungen: **DE - A - 2 437 297**

(73) Patentinhaber: **BASF Aktiengesellschaft, Carl-Bosch-Strasse 38, D-6700 Ludwigshafen (DE)**

(72) Erfinder: **Hagen, Helmut, Dr., Max-Slevogt-Strasse 17E, D-6710 Frankenthal (DE)**
Erfinder: **Markert, Juergen, Dr., Am Speyerweg 26, D-6704 Mutterstadt (DE)**
Erfinder: **Wuerzer, Bruno, Dr.Dipl.-Landw., Ruedigerstrasse 13, D-6701 Otterstadt (DE)**

**EP 0 060 429 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Dichlorchinolinderivate, ihre Herstellung, ihre Verwendung als Herbizide und Mittel dafür

Die vorliegende Erfindung betrifft Dichlorchinolinderivate, Verfahren zu deren Herstellung, Herbizide, die diese Verbindungen enthalten, sowie Verfahren zur Bekämpfung unerwünschten Pflanzenwuchses mit diesen Verbindungen.

Chinolinverbindungen, wie z.B. das 7-Chlorchinolin (DE-OS 23 22 143) und das 4-Chlor-3-nitrochinolin (US-PS 2 661 276) sind als Herbizide mit selektiven Eigenschaften sowie auch für den Bereich der totalen Vegetationsbekämpfung beschrieben. Bemerkenswert ist dabei die Abstufung der Wirkung, wonach das Einführen der Carboxylgruppe, z.B. bei der 7-Chlor-4-hydroxychinolin-2-carbonsäure im Vergleich zu 7-Chlor-4-hydroxychinolin und anderen Derivaten dieses Typs zu einem erheblichen Aktivitätsverlust führt (US-PS 2 661 276).

Es wurde nun gefunden, dass Dichlorchinolinderivate der Formel I

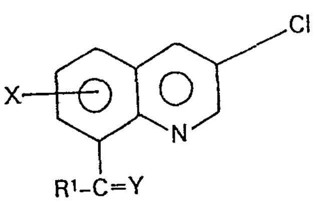

I,

in der

X für Chlor in den Positionen 5, 6 oder 7 steht,

Y für Sauerstoff, Schwefel, Hydroxyimino, zwei Wasserstoffatome, zwei Chloratome oder die Gruppe =N–A–B steht, worin A eine direkte Bindung oder eine $CH_2$-Gruppe und B einen gegebenenfalls durch Chloratome, Nitro-, Methyl- oder Trifluormethyl- oder Methoxygruppen substituierten Phenyl- oder Pyridinrest bedeuten, und $R^1$ für Wasserstoff, Halogen, Cyano, die Gruppe $-NR^2R^3$ – worin $R^2$ und $R^3$ gleich oder verschieden sind und Wasserstoff, $C_{1-6}$-Alkyl, $C_{1-6}$-Alkenyl, $C_{1-6}$-Hydroxyalkyl, Formyl, Cyclohexyl, Phenyl, Pyridinyl oder beide zusammen den Rest $(CH_2)_4$ oder $(CH_2)_5$, wobei eine $CH_2$-Gruppe durch ein Sauerstoff- oder Stickstoffatom oder eine $N-CH_3$-Gruppe ersetzt sein kann, bedeuten –, eine COOH-Gruppe oder die Gruppe OM steht, worin M ein Metall der 1. und 2. Hauptgruppe des Periodensystems, Wasserstoff, $C_{1-8}$-Alkyl, einen gebenenfalls durch Halogen, Nitro, $C_{1-4}$-Alkyl oder Trihalogenmethyl substituierten Phenylrest oder den Rest $H_2\overset{\oplus}{N}R^2R^3$, in dem $R^2$ und $R^3$ die oben angegebenen Bedeutung haben, darstellt oder Y und $R^1$ zusammen mit dem C-Atom eine Nitrilgruppe bedeuten,

eine gute herbizide Wirkung besitzen.

Vorzugsweise bedeutet in der Formel I X ein Chloratom in der 7-Stellung, $R^1$ ein Wasserstoffatom oder eine OH-, ONa-, $O\overset{\oplus}{N}H_2(CH_3)_2$- oder NHCHO-Gruppe und Y ein Sauerstoffatom, zwei Chlor- oder Wasserstoffatome oder die Gruppe

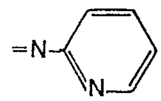

Die neuen Verbindungen werden dadurch hergestellt, dass man eine Verbindung der Formel II

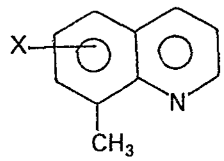

II,

worin X die oben genannte Bedeutung besitzt, mit einem Radikalstarter unter Ausschluss von Licht bei 140–190°C chloriert und die so erhaltenen Verbindungen der Formel III

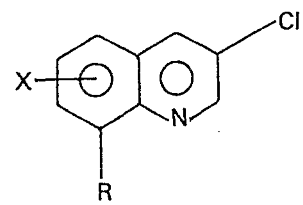

III,

worin X dasselbe wie oben und R Chlormethyl, Dichlormethyl oder Trichlormethyl bedeutet, gegebenenfalls in die Verbindungen der Formel I überführt, in denen der Rest $R^1-C=Y$ eine andere Bedeutung als R besitzt.

Die beim erfindungsgemässen Verfahren verwendeten 8-Methylchinoline sind bekannte Verbindungen. Die Herstellung ist z.B. von Bradford in J. Chem. Soc. 1947, S. 437, oder von Irving in J. Amer. Chem. Soc. 72, (1950), S. 4059 beschrieben.

Die Chlorierung von N-haltigen Heterozyklen erfolgt nach dem Stand der Technik in Essigsäure oder Schwefelsäure (vgl. Houben-Weyl, Bd. V/3, S. 725 f, Georg Thieme Verlag 1962) und bei Seitenkettenchlorierung unter gleichzeitiger Verwendung von UV-Licht, Houben-Weyl, Bd. V/3, S. 747, Georg Thieme Verlag 1962.

Überraschenderweise gelingt es, bei der Chlorierung der 8-Methylchinoline durch geeignete Wahl der Reaktionsparameter gleichzeitig eine spezifische Kernchlorierung in 3-Position der Chinoline sowie eine Seitenkettenchlorierung durchzuführen. Durch die Menge bzw. Dauer der Chlorzuführung ist bei den gewählten Reaktionsparametern eine exakte Reaktionsführung möglich, welche die gezielte Darstellung der Chlormethyl-, Dichlormethyl- bzw. Trichlormethylverbindungen erlaubt.

Die Chlorierung wird in einem inerten Lösungsmittel, wie z.B. Dichlorbenzolen oder Trichlorbenzolen, in Anwesenheit eines Radikalstarters, wie z.B. Azoisobutyronitril oder Benzoylperoxid, unter Ausschluss von Licht, in einem Temperaturbereich von 140–190°C, vorzugsweise bei

150–160°C durchgeführt. Bei Temperaturen oberhalb 190°C wird keine Kernchlorierung mehr erhalten.

Die Verbindungen, in denen Y ein Sauerstoffatom darstellt, erhält man durch Behandlung der entsprechenden Dichlorverbindungen mit starken Säuren, wie konzentrierte Schwefelsäure oder konzentrierte Salzsäure bei Temperaturen von 50–150°C.

Die Verbindungen, in denen Y ein Schwefelatom bedeutet, erhält man durch Umsetzen der entsprechenden Nitrile mit Schwefelwasserstoff in einem basischen Lösungsmittel, vorzugsweise Pyridin.

Die Verbindungen, in denen Y eine Hydroxyiminogruppe darstellt, erhält man aus den entsprechenden Aldehyden durch Erhitzen der entsprechenden Aldehyde mit Hydroxylamin bei 50–150°C. Verwendet man anstelle des freien Hydroxylamins salzsaures oder schwefelsaures Hydroxylamin, so muss eine Base zugegeben werden. Als Basen eignen sich Natriumcarbonat, Natriumhydrogencarbonat, Natronlauge oder die entsprechenden Kaliumverbindungen.

Die Verbindungen, in denen Y die Gruppe =N–A–B darstellt, erhält man aus den entsprechenden Aldehyden und den Verbindungen $H_2N$–A–B durch Erwärmen in Lösungsmitteln, wie Alkoholen und aliphatische Ether, die auch cyclisch sein können, auf 50–150°C.

Die Verbindungen, in denen $R^1$ eine Cyanogruppe ist, erhält man durch Umsetzen der entsprechenden Chlorverbindungen mit Kaliumcyanid oder Natriumcyanid in einem Lösungsmittel, wie Dimethylformamid oder Dimethylsulfoxid auf 100–200°C in Gegenwart von etwas Kaliumiodid.

Die Verbindungen, in denen $R^1$ die Gruppe $NR^2R^3$ darstellt, erhält man durch Reaktion der entsprechenden Amide mit der entsprechenden Chlorverbindung bei 30–100°C in An- oder Abwesenheit von Lösungsmitteln, wie Alkoholen, Ethern oder Dimethylsulfoxid.

Die Ammonium-Verbindungen erhält man durch Umsetzung der entsprechenden Carbonsäuren mit Aminen, beispielsweise in Alkohol, Dimethylformamid oder Dimethylsulfoxid bei 50–150°C.

Die Verbindungen, in denen $R^1$ eine COOH-Gruppe ist, erhält man z.B. durch Verseifen der entsprechenden Nitrile mit konzentrierter Schwefelsäure.

Die Verbindungen, in denen $R^1$ für OH steht, erhält man durch Umsetzen der entsprechenden Chlorverbindungen mit Natrium- oder Kaliumhydroxid.

Die folgenden Beispiele erläutern die Herstellung der neuen Verbindungen:

Beispiel 1

89 Teile 7-Chlor-8-methylchinolin und 0,5 Teile Azo-bisisobutyronitril werden in 500 Teilen Dichlorbenzol vorgelegt und auf 140°C erhitzt. Bei dieser Temperatur wird mit dem Einleiten von 80 Teilen Chlor begonnen. Während des Einleitens von Chlor wird die Temperatur auf 160°C erhöht. Nach Beendigung der Chlorzugabe wird die Lösung mit Stickstoff gespült, der grösste Teil des Lösungsmittels abdestilliert, der ausgefallene Feststoff abgesaugt und mit Petrolether gewaschen. Es werden 113 Teile 3,7 Dichlor-8-chlormethylchinolin vom Schmp. 129°C erhalten. Die Ausbeute entspricht 93% der Theorie.

Beispiel 2

177 Teile 7-Chlor-8-methylchinolin und 1 Teil Azo-bisisobutyronitril werden in 1000 Teilen Dichlorbenzol vorgelegt und auf 140°C erhitzt. Bei dieser Temperatur wird mit dem Einleiten von 250 Teilen Chlor begonnen. Die Temperatur wird auf 175°C erhöht. Nach erfolgter Reaktion wird die Lösung mit Stickstoff gespült, anschliessend der grösste Teil des Lösungsmittels abdestilliert, der ausgefallene Feststoff abgesaugt und mit Petrolether gewaschen. Man erhält 255 Teile 3,7-Dichlor-8-dichlormethylchinolin vom Schmp. 154°C. Die Ausbeute entspricht 80% der Theorie.

Beispiel 3

35 Teile 6-Chlor-8-methylchinolin und 0,5 Teile Azo-bisisobutyronitril werden in 200 Teilen Dichlorbenzol auf 140°C erhitzt. Bei dieser Temperatur wird mit dem Einleiten von 70–90 Teilen Chlor begonnen, während die Temperatur auf 180°C erhöht wird. Nach Beendigung der Reaktion wird die Lösung mit Stickstoff gespült, das Lösungsmittel abdestilliert und der Rückstand aus Ligroin umkristallisiert. Es werden 52 Teile 3,6-Dichlor-8-trichlormethylchinolin vom Schmp. 198°C erhalten. Die Ausbeute entspricht 82% der Theorie.

Beispiel 4

56 Teile 3,7-Dichlor-8-dichlormethylchinolin werden in 250 Teilen 90%iger Schwefelsäure 6 Stunden bei 100°C gerührt. Die Lösung wird nach dem Abkühlen auf Eis gegossen, der ausgefallene Feststoff abgesaugt, mit Wasser neutral gewaschen und getrocknet. Man erhält 195 Teile 3,7-Dichlor-8-chinolincarbaldehyd vom Schmp. 208°C. Die Ausbeute beträgt 87% der Theorie.

Beispiel 5

22,6 Teile 3,7-Dichlor-8-chinolincarbaldehyd (Beispiel 4) werden in 300 Teilen Alkohol gelöst. Zu dieser Lösung werden 10,6 Teile Natriumcarbonat und 6,9 Teile Hydroxylammoniumchlorid hinzugefügt und die Suspension 1 Stunde am Rückfluss erhitzt. Anschliessend versetzt man mit 1000 Teilen Wasser, filtriert den ausgefallenen Feststoff ab und trocknet ihn. Es werden 23 Teile 3,7-Dichlor-8-hydroxyliminochinolin vom Schmp. 202°C erhalten. Die Ausbeute entspricht 96% der Theorie.

Beispiel 6

24,6 Teile 3,7-Dichlor-8-chlormethylchinolin und 30 Teile Diethylamin werden 6 Stunden bei 55°C gerührt. Man versetzt mit Wasser, saugt den Feststoff ab und trocknet ihn. Es werden 25 Teile 3,7-Dichlor-8-diethylaminomethylchinolin

vom Schmp. 54°C erhalten. Die Ausbeute beträgt 89% der Theorie.

Beispiel 7

24,6 Teile 3,7-Dichlor-8-chlormethylchinolin (Beispiel 1) und 15 Teile Natrium-p-chlorphenolat werden in 200 Teilen Dimethylformamid 10 Stunden bei 100°C gerührt. Das Lösungsmittel wird abdestilliert, der Rückstand mit Wasser versetzt, abgesaugt, getrocknet und aus einem geeigneten Lösungsmittel umkristallisiert. Man erhält 32 Teile 3,7-Dichlor-8-p-chlorphenoxymethylchinolin vom Schmp. 110°C. Die Ausbeute entspricht 95% der Theorie.

Beispiel 8

49 Teile 3,7-Dichlor-8-chlormethylchinolin (Beispiel 1), 13 Teile Kaliumcyanid und 0,1 Teil Kaliumjodid werden in 300 Teilen Dimethylformamid 4 Stunden auf 150°C erhitzt. Die Reaktionslösung wird mit Wasser versetzt, das ausgefallene Produkt abgesaugt und aus Methylglykol umkristallisiert. Es werden 32 Teile 3,7-Dichlor-8-cyanomethylchinolin vom Schmp. 152°C erhalten. Dies entspricht einer Ausbeute von 67% der Theorie.

Beispiel 9

28,1 Teile 3,7-Dichlor-8-chlormethylchinolin (Beispiel 1), 6,95 Teile Hydroxylaminohydrochlorid und 13,6 Teile Natriumformiat werden in 200 ml Ameisensäure und 60 ml Wasser 12 Stunden bei 100°C gerührt. Die Reaktionslösung wird auf Eis gegossen, der ausgefallene Feststoff abgesaugt, mit Wasser neutral gewaschen und getrocknet. Man erhält 19 Teile 3,6-Dichlor-8-cyanochinolin vom Schmp. 222°C. Das entspricht einer Ausbeute von 78,5% der Theorie.

Beispiel 10

200 Teile 3,7-Dichlor-8-cyanochinolin (Beispiel 9) werden in 2600 Teilen 65%iger Schwefelsäure 20 Stunden bei 140°C gerührt. Die abgekühlte Lösung wird auf Eis gegossen, der ausgefallene Feststoff abgesaugt, getrocknet, in Dimethylformamid aufgenommen, mit Aktivkohle versetzt, filtriert, mit Wasser versetzt, abgesaugt, mit Wasser gewaschen und getrocknet. Es werden 200 Teile 3,7-Dichlor-8-chinolincarbonsäure vom Schmp. 272°C erhalten. Die Ausbeute beträgt 92% der Theorie.

Beispiel 11

20 Teile 3,5-Dichlor-8-chinolincarbaldehyd (hergestellt analog Beispiel 4) und 14,6 Teile 2,4-Dichloranilin werden in 300 Teilen Alkohol 3 Stunden am Rückfluss erhitzt. Die Lösung wird abgekühlt, das ausgefallene Produkt abgesaugt und aus Methylglykol umkristallisiert. Man erhält 30 g 3,5-Dichlor-8-(2,4-dichlorphenyliminomethyl)-chinolin vom Schmp. 156°C. Die Ausbeute beträgt 90% der Theorie.

Beispiel 12

24,6 Teile 3,7-Dichlor-8-chlormethylchinolin (Beispiel 1) werden in 300 Teilen Formamid 5 Stunden am Rückfluss erhitzt. Man versetzt mit Wasser, saugt den Feststoff ab und kristallisiert aus Methanol um. Es werden 23 Teile 3,7-Dichlor-8-formylaminomethylchinolin vom Schmp. 110°C erhalten. Die Ausbeute beträgt 94% der Theorie.

Beispiel 13

22 Teile 3,6-Dichlor-8-cyanochinolin (hergestellt analog Beispiel 8) werden in 80 Teilen Triethylamin und 80 Teilen Pyridin auf 40°C erhitzt, 4 Stunden lang wird langsam $H_2S$-Gas eingeleitet. Anschliessend wird mit Wasser versetzt, der Feststoff abgesaugt, mit Wasser und Aceton gewaschen und aus Essigester umkristallisiert. Man erhält 17 Teile 3,6-Dichlor-8-chinolinthiocarbonsäureamid vom Schmp. 226°C. Die Ausbeute entspricht 66% der Theorie.

Analog Beispielen 1–12 werden hergestellt:

| Beispiel | X | Y | $R^1$ | Schmp. [°C] |
|---|---|---|---|---|
| 14 | 7-Cl | =N-(2-pyridyl) | H | 104 |
| 15 | 7-Cl | O | 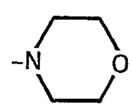 | 206 |
| 16 | 7-Cl | O | $OC_3H_7$ | 112 |
| 17 | 7-Cl | H,H | O-(4-$CH_3C_6H_4$) | 88-90 |
| 18 | 7-Cl | H,H | O-(2,4-Di$NO_2$-$C_6H_3$) | 144 |
| 19 | 7-Cl | H,H | O-(2,4-DiCl-$C_6H_3$) | 134 |
| 20 | 7-Cl | H,H | O-(4-$CH_3O$-$C_6H_4$) | 155 |
| 21 | 7-Cl | H,H | O-(2-$NO_2$-$C_6H_4$) | 167–168 |
| 22 | 7-Cl | H,H | O-(3-$NO_2$-$C_6H_4$) | 140–142 |

Analog Beispielen 1-12 werden hergestellt:

| Beispiel | X | Y | $R^1$ | Schmp. [°C] |
|---|---|---|---|---|
| 23 | 7-Cl | H,H | $O-(C_6H_5)$ | – |
| 24 | 7-Cl | H,H | $O-(4-Br-C_6H_4)$ | – |
| 25 | 7-Cl | H,H | $O-(4-CCl_3-C_6H_4)$ | – |
| 26 | 7-Cl | H,H | $OC_4H_9$ | – |
| 27 | 7-Cl | H,H | $OC_5H_{11}$ | – |
| 28 | 7-Cl | H,H | $O_6H_{13}$ | – |
| 29 | 7-Cl | H,H | $OCH_3$ | 78 |
| 30 | 7-Cl | H,H | $OC_2H_5$ | – |
| 31 | 7-Cl | H,H | $O-iso-C_3H_7$ | – |
| 32 | 7-Cl | O | $N(C_2H_5)_2$ | 145 (.3 $H_2O$) |
| 33 | 7-Cl | O | 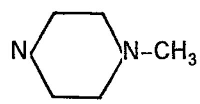 | 223 |
| 34 | 7-Cl | O | $NH_2$ | 238 |
| 35 | 7-Cl | O | $N(CH_2-CH_2OH)_2$ | – |
| 36 | 7-Cl | O | $N(CH_2-CH=CH_2)_2$ | – |
| 37 | 7-Cl | O | 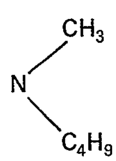 | |
| 38 | 7-Cl | O | 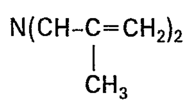 | – |
| 39 | 7-Cl | O | N-piperidinyl | – |
| 40 | 7-Cl | O | N-pyrrolidinyl | – |
| 41 | 7-Cl | O | $NHCH_3$ | – |
| 42 | 7-Cl | O | $NH-C_6H_5$ | – |
| 43 | 7-Cl | S | $NH_2$ | – |
| 44 | 7-Cl | S | $N(CH_2CH_2OH)_2$ | – |
| 45 | 7-Cl | H,H | 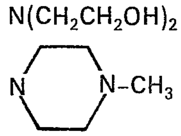 | 82 |
| 46 | 7-Cl | H,H | NH-(2-pyridyl) | >270 (. HCl) |
| 47 | 7-Cl | H,H | $NH-CH_2-CH=CH_2$ | 198 (. HCl) |

Analog Beispielen 1-12 werden hergestellt:

| Beispiel | X | Y | $R^1$ | Schmp. [°C] |
|---|---|---|---|---|
| 48 | 7-Cl | H,H | 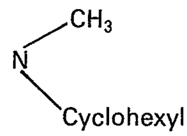 | 230 (. HCl) |
| 49 | 7-Cl | H,H | $N(C_3H_7)_2$ | 84 (. HCl) |
| 50 | 7-Cl | H,H | $N(Cyclohexyl)_2$ | >280 (. HCl) |
| 51 | 7-Cl | H,H | $NH-C_6H_{13}$ | 110 (. HCl) |
| 52 | 7-Cl | H,H | N-pyrrolidinyl | 260 (. HCl) |
| 53 | 7-Cl | H,H | N-piperidinyl | >260 (. HCl) |
| 54 | 7-Cl | H,H | 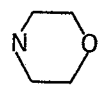 | 117 |
| 55 | 7-Cl | H,H | 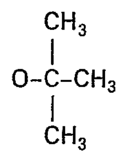 | 90 |
| 56 | 7-Cl | O | $OCH_3$ | 102 |
| 57 | 7-Cl | O | $OC_2H_5$ | 109 |
| 58 | 7-Cl | O | $OC_4H_9$ | - |
| 59 | 7-Cl | O | $OC_5H_{11}$ | - |
| 60 | 7-Cl | O | $OC_6H_{13}$ | - |
| 61 | 6-Cl | $=N-(4-Cl-C_6H_4)$ | H | 180 |
| 62 | 6-Cl | $=N-(2,4-DiCl-C_6H_3)$ | H | 176 |
| 63 | 6-Cl | $=N-(2,5-DiCH_3-C_6H_3)$ | H | 140 |
| 64 | 5-Cl | $=N-(3CF_3-C_6H_4)$ | H | 160 |
| 65 | 6-Cl | $=N-(2,4-DiCH_3-C_6H_3)$ | H | 130 |
| 66 | 5-Cl | $=N-(2,4-DiCH_3-C_6H_3)$ | H | 120 |
| 67 | 6-Cl | 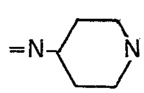 | H | 180 |
| 68 | 5-Cl | $=N-(4-NO_2-C_6H_4)$ | H | 260 |
| 69 | 7-Cl | $=N-CH_2-(2,6-DiCH_3-C_6H_3)$ | H | 154 |
| 70 | 6-Cl | $=N-(4-CH_3O-C_6H_4)$ | H | 180 |
| 71 | 6-Cl | Cl, Cl | H | 134 |
| 72 | 6-Cl | O | OH | 238 |

Analog Beispielen 1–12 werden hergestellt:

| Beispiel | X | Y | $R^1$ | Schmp. [°C] |
|---|---|---|---|---|
| 73 | 5-Cl | O | $\overset{\ominus\oplus}{ONa}$ | > 300 |
| 74 | 7-Cl | O | $\overset{\ominus\oplus}{ONa}$ | > 300 |
| 75 | 7-Cl | O | $\overset{\ominus\oplus}{O}NH_2(CH_3)_2$ | 180 |
| 76 | 5-Cl | Cl, Cl | H | 130 |
| 77 | 5-Cl | O | H | 170 |
| 78 | 7-Cl | H,H | COOH | 210 |
| 79 | 5-Cl | O | OH | 200 |
| 80 | 5-Cl | O | $\overset{\ominus\oplus}{O}NH_2(CH_3)_2$ | > 270 |
| 81 | 5-Cl | O | Cl | – |
| 82 | 6-Cl | O | Cl | 75 |
| 83 | 5-Cl | O | $OCH_3$ | – |
| 84 | 5-Cl | O | $OC_4H_9$ | – |
| 85 | 5-Cl | O | $OC_6H_{13}$ | – |
| 86 | 7-Cl | O | $OCH_2CH(C_2H_5)C_4H_9$ | – |
| 87 | 7-Cl | Cl, Cl | Cl | 135 |
| 88 | 7-Cl | O | Cl | 91 |
| 89 | 6-Cl | O | $\overset{\ominus\oplus}{ONa}$ | > 300 |
| 90 | 6-Cl | $=N-(3-CF_3-C_6H_4)$ | H | 103 |
| 91 | 6-Cl | O | $OCH_3$ | – |
| 92 | 6-Cl | O | $OC_5H_{11}$ | – |
| 93 | 6-Cl | O | $OC_3H_7$ | – |
| 94 | 6-Cl | O | $N(C_2H_5)_2$ | – |
| 95 | 5-Cl | O | 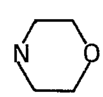 | – |
| 96 | 5-Cl | O | 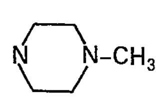 | – |
| 97 | 5-Cl | O | $N(CH_3)_2$ | – |
| 98 | 6-Cl | O | N-pyrrolidinyl | – |
| 99 | 6-Cl | N | | 150 |
| 100 | 5-Cl | N | | 174 |

Die Wirkung von Vertretern der neuen substituierten Chinolinderivate auf das Wachstum von erwünschten und unerwünschten Pflanzen wird anhand von Gewächshausversuchen vorgeführt:

Als Kulturgefässe dienten Plastikblumentöpfe mit 300 $cm^3$ Inhalt und lehmigem Sand mit etwa 1,5% Humus als Substrat. Bei Galium aparine wurde zusätzlich etwas Torfmull beigemischt, ebenso bei dem für die Nachauflaufbehandlung angezogenen Reis, um ein einwandfreies Wachstum zu gewährleisten. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich je nach Wirkstoff um 0,5, 1,0 oder 2,0 kg/ha. Nach dem Aufbringen der Mittel wurden die Gefässe leicht beregnet, um Keimung und Wachstum in Gang zu bringen. Danach deckte man die Gefässe mit durchsichtigen Plastikhauben ab, bis die Pflanzen angewachsen waren. Diese Abdeckung bewirkte ein gleichmässiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 15 cm an und behandelte sie danach. Zur Nachauflaufbehandlung wurden entweder direkt gesäte und in den gleichen Gefässen aufgewachsene Pflanzen ausgewählt, oder sie wurden erst als Keimpflanzen getrennt angezogen und einige Tage vor der Behandlung in die Versuchsgefässe verpflanzt. Die Aufwandmengen für die Nachauflaufbehandlung waren ebenfalls je nach Verbindung unterschiedlich und betrugen 1,0, 2,0 und 4,0 kg/ha Wirkstoff. Die Abdeckung unterblieb bei der Nachauflaufbehandlung. Die Aufstellung der Versuche erfolgte im Gewächshaus, wobei für wärmeliebende Arten wärmere Bereiche (20 bis 30°C) und für solche gemässigter Klimate 15 bis 25°C bevorzugt wurden. Die Versuchsperiode erstreckte sich über 3 bis 4 Wochen.

Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Zu Vergleichszwecken dienten unter denselben Versuchsbedingungen bei Vor- und Nachlaufanwendung die bekannten Verbindungen 7-Chlorchinolin (A) (DE-OS 2 322 143) und 7-Chlor-4-hydrochinolin-2-carbonsäure (B) (US-PS 2 661 276), deren Aufwandmenge 1,0 kg/ha betrug.

Die Ergebnisse zeigen, dass die Verbindungen bei Vor- und Nachauflaufanwendung eine beachtliche herbizide Wirkung haben und gleichzeitig für verschiedene Kulturpflanzen als selektive Mittel verträglich sind oder gegebenenfalls nur geringfügige Schäden verursachen.

Sind gewisse Kulturpflanzen gegenüber den Wirkstoffen etwas empfindlich, so können auch Ausbringungstechniken angewandt werden, bei welchen die herbiziden Mittel mit Hilfe der Spritzgeräte so geleitet werden, dass die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post-directed, lay-by). In Anbetracht der guten Verträglichkeit und der Vielseitigkeit der Applikationsmethoden können die erfindungsgemässen Herbizide noch in einer weiteren grossen Zahl von Kulturpflanzen zur Beseitigung unerwünschten Pflanzenwuchses eingesetzt werden. Die Aufwandmengen können dabei zwischen 0,05 und 10 kg/ha und mehr schwanken.

In Betracht kommen beispielsweise folgende Kulturen:

| Botanischer Name | Deutscher Name | Englischer Name |
|---|---|---|
| Abutilon theophrasti | Chinesischer Hanf | velvet leaf |
| Allium cepa | Küchenzwiebel | onions |
| Ananas comosus | Ananas | pineapple |
| Arachis hypogaea | Erdnuss | peanuts (groundnuts) |
| Asparagus officinalis | Spargel | asparagus |
| Avena sativa | Hafer | oats |
| Beta vulgaris | Zuckerrübe | sugarbeets |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weisse Rübe | turnips |
| Brassica rapa var. silvestris | Rübsen | |
| Camellia sinensis | Teestrauch | tea plants |
| Carthamus tinctorius | Saflor — Färberdistel | safflower |
| Carya illinoinensis | Pekannussbaum | pecan trees |
| Cirsium arvense | Acker-Kratzdistel | Canada thistle |
| Citrus limon | Zitrone | lemon |
| Citrus maxima | Pampelmuse | grapefruits |

| Botanischer Name | Deutscher Name | Englischer Name |
|---|---|---|
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süsskartoffeln | sweet potato |
| Juglans regia | Walnussbaum | walnut trees |
| Lactuca sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weisstanne | fire |
| Pinus spp. | Kiefer | pine trees |
| Pisum sativum | Gartenerbse | English peas |
| Prunus avium | Süsskirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |

| Botanischer Name | Deutscher Name | Englischer Name |
|---|---|---|
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preisselbeere | cranberry |
| Veronica persica | Persischer Ehrenpreis | |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Zur Verbreiterung des Wirkungsspektrums und zur Erzielung synergistischer Effekte können die neuen substituierten Chinolinderivate sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-(3-trifluormethylphenyl)-3(2H)-pyridazinon
5-Methylamino-4-chlor-2-(3-α,α,β,β-tetrafluorethoxyphenyl)-3-(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3-trifluormethylphenyl)-3(2H)-pyridazinon
5-Methoxy-4-chlor-2-(3-trifluormethylphenyl)-3(2H)-pyridazinon
5-Amino-4-brom-2-(3-methylphenyl)-3(2H)-pyridazinon

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-methylethyl)-1H-(pyridino[3,2-e]2,1,3-thiadiazin-4)-on-2,2-dioxid

N-(1-Ethylpropyl)-2,6-dinitro-3,4-dimethylanilin
N-(1-Methylethyl)-N-ethyl-2,6-dinitro-4-trifluormethyl-anilin
N-n.Propyl-N-β-chlorethyl-2,6-dinitro-4-trifluormethyl-anilin
N-n.Propyl-N-cyclopropylmethyl-2,6-dinitro-4-trifluor-methyl-anilin
N-Bis(n.propyl)-2,6-dinitro-3-amino-4-trifluormethylanilin
N-Bis(n.propyl)-2,6-dinitro-4-methyl-anilin
N-Bis(n.propyl)-2,6-dinitro-4-methylsulfonyl-anilin
N-bis(n.propyl)-2,6-dinitro-4-aminosulfonyl-anilin
Bis(β-chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di(tert.butyl)-4-methylphenyl-ester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim

N-Ethyl-2-(phenylcarbamoyl)-oxypropionsäure-amid
3'-N-Isopropyl-carbamoyloxy-propionanilid

Ethyl-N-(3-(N'-phenylcarbamoyloxy)-phenyl)-carbamat
Methyl-N-(3-(N'-methyl-N'-phenylcarbamoyl-oxy)-phenyl)-carbamat
Isopropyl-N-(3-(N'-ethyl-N'-phenylcarbamoyl-oxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-N'-3-chlor-4-fluorphenylcarbamoyl-oxy)-phenyl)-carbamat
Ethyl-N-(3-N'-3,4-difluorphenylcarbamoyloxy)-phenyl)-carbamat
Methyl-N-(3-(N'-3,4-difluorphenylcarbamoyl-oxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-ethylester
N-3-(4-Fluorphenoxycarbonylamino)-phenyl-thiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäure-methylester

N,N-Diethyl-thiolcarbaminsäure-p-chlorbenzyl-ester
N,N-Di n-propyl-thiolcarbaminsäure-ethylester
N,N-Di n-propyl-thiolcarbaminsäure-n-propyl-ester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec. butyl-thiolcarbaminsäure-ethylester
N,N-Di-sec. butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäure-ethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat

N-Ethyl-N-n-butyl-thiolcarbaminsäure-n.propyl-ester
N,N-Dimethyl-dithiocarbaminsäure-2-chlorallyl-ester
N-Methyl-dithiocarbaminsäure-Natrium
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl,α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäure-isobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäure-ethylester
2-(N-Benzoyl-3-chlor-4-fluorphenylamino)-propionsäure-methylester
2-(N-Benzoyl-3-chlor-4-fluorphenylamino)-propionsäure-isopropylester

2-Chlor-4-ethylamino-6-isoproylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin

2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-2(-Tetrahydropyranyl)-5,6-trimethylenuracil
3-Cyclohexyl-5,6-trimethylenuracil

2-Methyl-4-(3′-trifluormethylphenyl)-tetrahydro-1,2,4-oxadiazin-3,5-dion
2-Methyl-4-(4′-fluorphenyl)-tetrahydro-1,2,4-oxadiazin-3,5-dion
3-Amino-1,2,4-triazol
1-Allyloxy-1-(4-bromphenyl)-2-[1′,2′,4′-triazolyl-(1′)]-ethan (Salze)
1-(4-Chlorphenoxy)-3,3-dimethyl-1-(1H-1,2,4-triazol-1-yl)-2-butanon
N,N-Diallylchloracetamid
N-Isopropyl-2-chloracetanilid
N-(1-Methyl-propin-2-yl)-2-chloracetamid

2-Methyl-6-ethyl-N-(propargyl)-2-chloracetanilid
2-Methyl-6-ethyl-N-(äthoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4,-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid

2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n.butoxymethyl)-2-chloracetanilid
2,6-Diethyl-N-(ethoxycarbonylmethyl)-2-chloracetanilid
2,3,6-Trimethyl-N-(pyrazol-1-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl)-4-chlorphenoxy-N-methoxy-acetamid
2-(α-Naphthoxy)-N,N-diethylpropionamid
2,2-Diphenyl-N,N-dimethylacetamid
α-(3,4,5-Tribrompyrazol-1-yl)-N,N-dimethylpropionamid
N-(1,1-Dimethylpropionyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethansulfonanilid
5-Acetamido-4-methyl-trifluormethan-sulfonanilid

2-Propionyl-amino-4-methyl-5-chlor-thiazol
O-(Methylsulfonyl)-glykolsäure-N-ethoxymethyl-2,6-dimethyl-anilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenamid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
3,5-Dibrom-4-hydroxy-O-2-Cyan-4-nitrophenylbenzaldoxim (Salze)
Pentachlorphenyl-Natriumsalz
2,4-Dichlorphenyl-4′-nitrophenylether
2,4,6-Trichlorphenyl-4′-nitrophenylether
2-Fluor-4,6-dichlorphenyl-4′-nitrophenylether
2-Chlor-4-trifluormethylphenyl-4′-nitrophenylether
2,4′-Dinitro-4-trifluormethyl-diphenylether
2,4-Dichlorphenyl-3′-methoxy-4′-nitro-phenylether
2-Chlor-4-trifluormethylphenyl-3′-ethoxy-4′-nitro-phenylether
2-Chlor-4-trifluormethylphenyl-3′-carboxy-4′-nitro-phenylether (Salze)
2,4-Dichlorphenyl-3′-methoxycarbonyl-4′-nitrophenylether
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion

2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methan-sulfonat
2-Ethoxy-2,3-dihydro-3,3-dimethyl-5-benzofuranyl-dimethyl-aminosulfonat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec.Butyl-4,6-dinitrophenol (Salze, Ester)
2-sec.Butyl-4,6-dinitrophenol-acetat
2-tert.Butyl-4,6-dinitrophenol-acetat

2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat

2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(α,α,β,β-Tetrafluoräthoxyphenyl)-3,3-dimethyl-harnstoff

1-(3-tert.Butylcarbamoyloxy-phenyl)-3,3-dimethyl-harnstoff
1-(3-Chlor-4-methylphenyl)-3,3-dimethyl-harnstoff
1-(3-Chor-4-methoxyphenyl)-3,3-dimethyl-harnstoff
1-(3,5-Dichlor-4-methoxyphenyl)-3,3-dimethyl-harnstoff
1-[4-(4′-Chlorphenoxy)-phenyl]-3,3-dimethyl-harnstoff
1-[4-(4′-methoxyphenoxy)-phenyl]-3,3-dimethyl-harnstoff
1-Cyclooktyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-iso-butylamid
1,2-Dimethyl-3,5-diphenylpyrazolium-methylsulfat
1,2-4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenyl-sulfonyl)-oxy]-pyrazol
2,3,5-Trichlor-pyridinol-(4)
1-Methyl-3-phenyl-5-(3′-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1′-Dimethyl-4,4′-dipyridylium-di-(methylsulfat)
1,1′-Di(3,5-dimethylmorpholin-carbonylmethyl)-4,4′-di-pyridylium-dichlorid
1,1′-Ethylen-2,2′-dipyridylium-dibromid
3-[1-(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxy-carbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)

2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy-acrylat)
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis-(phosphonmethyl)-glycin (Salze)
2-Chlorethanphosphonsäure-2-chlorethylester
Ammonium-ethyl-carbamoyl-phosphonat
Di-n.butyl-1-n.butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
Natriumchlorat
Ammoniumrhodanid
Calciumcyanamid
2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Iodpyridyl-2-oxy)-phenoxy]-propionsäureethylester
2-[4-(5'-Iodpyridyl-2-oxy)-phenoxy]-propionsäure-n-butylester
2-Chlor-4-trifluormethylphenyl-3'-(2-fluorethoxy)-4'-nitrophenylether
2-Chlor-4-trifluormethylphenyl-3-(ethoxycarbonyl)-methylthio-4-nitrophenylether
2,4,6-Trichlorphenyl-3-(ethoxycarbonyl)-methylthio-4-nitrophenylether
2-[1-(N-ethoxamino)-butyliden]-5-(2-ethylthiopropyl)-3-hydroxy-cyclohexen-(2)-on-(1) (Salze)
2-[1-(N-ethoxamino)-butyliden]-5-(2-phenylthiopropyl)-3-hydroxy-cyclohexen-(2)-on-(1) (Salze)

4[4-(4'-Trifluormethyl)-phenoxy]-penten-2-carbonsäureethylester

2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-α,α,β-trifluor-β-bromoethoxyphenyl)-3-(2H)-pyridazinon
2,4-Dichlorphenyl-3'-ethoxy-ethoxy-ethoxy-4'-nitrophenylether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1-(3-Chlor-4-ethoxyphenyl)-3,3-dimethylharnstoff
2-Methyl-4-Chlorphenoxy-thioessigsäureethylester
2-Chlor-3,5-diiod-4-acetoxy-pyridin
1-(4-[2-(4-Methylphenyl)-ethoxy]-phenyl)-3-methyl-3-methoxyharnstoff
2,6-Dimethyl-N-(pyrazol-1-yl-methylenoxymethyl)-2-chloracetanilid
2-Methyl-6-ethyl-N-(pyrazol-1-yl-methylenoxymethyl)-2-chloracetanilid
1-(α-2,4-Dichlorphenoxypropionsäure)-3-(O-methylcarbamoyl)-anilid

1-(α-2-Brom-4-chlorphenoxypropionsäure)-3-(O-methyl-carbamoyl)-anilid
2-Methyl-6-ethyl-N-(pyrazol-1-yl-ethylenoxymethyl)-2-chloracetanilid
Methyl-N-dichlorfluormethylsulfenyl-(3-(N-dichlorfluormethylsulfenyl-N'-phenylcarbamoyl-oxy)-phenyl)-carbamat
Methyl-N-dichlorfluormethylsulfenyl-(3-N'-dichlorfluormethylsulfenyl-N'-3-methylphenylcarbamoyl-oxy)-phenyl)-carbamat
N-(Pyrazol-1-yl-methyl)-pyrazol-1-yl-essigsäure-2,6-dimethylanilid
N-(Pyrazol-1-yl-methyl)-1,2,4-triazol-1-yl-essigsäure-2,6-dimethylanilid
2-(3'-Trifluormethylphenyl)-4H-3,1-benzoxazin-4-on
2-(2-Thienyl)-4H-3,1-benzoxazin-4-on

Ausserdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Bei der Prüfung auf selektive herbizide Wirkung bei Vorauflaufanwendung im Gewächshaus bei Aufwandmengen von 2,0 kg Wirkstoff/ha zeigt z.B. die Verbindung des Beispiels 2 eine recht gute herbizide Wirkung und eine gute bzw. akzeptable Verträglichkeit für Reis, Sonnenblumen und Weizen. Ebenso hat diese Verbindung bei der selektiven Unkrautbekämpfung bei Nachauflaufanwendung im Gewächshaus bei Aufwandmengen von 2,0 kg Wirkstoff/ha eine beachtliche herbizide Wirkung mit guter oder annehmbarer Toleranz für Kulturpflanzen wie Hafer, Reis, Raps und Weizen.

Bei der Prüfung der herbiziden Wirkung bei Vorauflaufbehandlung im Gewächshaus bei der Aufwandmenge von 1,0 kg Wirkstoff/ha entfaltet z.B. die Verbindung des Beispiels 4 eine gute herbizide Wirkung bei gleichzeitig guter und brauchbarer Verträglichkeit für die als Beispiele angeführten Kulturpflanzen Raps, Reis und Kultursorghum.

Ferner erwies sich z.B. die Verbindung des Beispiels 14 bei der Prüfung der herbiziden Wirkung bei Vorauflaufanwendung im Gewächshaus bei der Aufwandmenge von beispielsweise 1,0 kg Wirkstoff/ha von guter herbizider Aktivität mit gleichzeitig durchgehend guter Selektivität für Raps, Reis und Weizen.

Ebenso zeigte die Verbindung des Beispiels 10 auf Vorauflaufanwendung im Gewächshaus bei der Beispielsaufwandmenge von 1,0 kg Wirkstoff/ha eine sehr gute herbizide Wirkung gegen grasartige und breitblättrige unerwünschte Pflanzen.

Bei der Prüfung der herbiziden Wirkung bei Nachauflaufbehandlung im Gewächshaus von 1,0 kg/ha hatte die Verbindung des Beispiels 10 ebenfalls eine sehr gute herbizide Aktivität bei einer gleichzeitig nur geringfügigen Hemmung des Wachstums von Kulturpflanzen, z.B. Reis. Derselbe Wirkstoff eignet sich je nach zu bekämpfenden Unkrautarten und entsprechend gewählten Dosierungen auch als selektives Mittel in anderen Kulturpflanzen.

Die Verbindung des Beispiels 75 erbrachte bei Vor- und Nachauflaufbehandlung mit 0,5 kg/ha bzw. 1,0 kg/ha gegen wichtige unerwünschte Pflanzen eine herbizide Wirkung bei gleichzeitig guter Verträglichkeit für Kulturpflanzen.

Desgleichen zeigte die Verbindung des Beispiels 74 bei Nachauflaufbehandlung im Gewächshaus bei der Aufwandmenge von 1,0 kg Wirkstoff/ha eine gute und selektive herbizide Wirkung.

Ferner bekämpfte die Verbindung des Beispiels 12 bei Nachauflaufwendung von 2,0 kg Wirkstoff/ha eine Reihe grasartiger und breitblättriger unerwünschter Pflanzen bei gleichzeitig guter Verträglichkeit für Kulturpflanzen.

In den beschriebenen Gewächshausversuchen erwies sich die Verbindung des Beispiels 10 bei Vor- und Nachauflaufanwendung von beispielsweise 1,0 kg Wirkstoff/ha in der herbiziden Aktivität den Vergleichsverbindungen A und B weit überlegen.

Bei der Prüfung der herbiziden Wirkung bei Nachauflaufanwendung im Gewächshaus hatten die Verbindungen 16 mit 2,0 kg/ha und Verbindung 1 mit 4,0 kg/ha eine recht beachtliche selektive herbizide Wirkung. Ebenso zeigten die Verbindungen 16 und 69 mit 2,0 kg/ha bei Vorauflaufanwendung im Gewächshaus eine selektive herbizide Wirkung.

Weiterhin konnte in Gewächshausversuchen am Beispiel von Azaleen (Rhododendron simsii) gezeigt werden, dass z.B. Verbindung 10 das Triebwachstum von verholzten Pflanzen reguliert und somit je nach Dosis als Wachstumsregulator oder zur Bekämpfung derselben infrage kommt.

Im Freiland erreichte Verbindung 10 bei Vorauflaufanwendung von 0,5 kg Wirkstoff/ha in Kleinparzellenversuchen auf Standorten mit lehmigem Sand mit pH 6 und 1 bis 1,5% Humus ebenfalls eine sehr gute Bekämpfung natürlich vorkommender Unkräuter, ohne Beispielskulturen wie Raps, Wintergerste oder Mais zu schädigen. Die Wirkstoffe wurden hierzu in Wasser als Träger- und Verteilermedium emulgiert oder suspendiert und mit Hilfe einer auf einen Traktor montierten Parzellenspritze ausgebracht. Bei Fehlen natürlicher Niederschläge beregnete man künstlich, um Keimung und Wachstum von Nutzpflanzen und Unkräutern zu gewährleisten. Die Bewertung erfolgte zu gewissen Abständen ebenfalls nach der Skala von 0 bis 100.

**Patentansprüche**

1. Dichlorchinolinderivate der Formel I

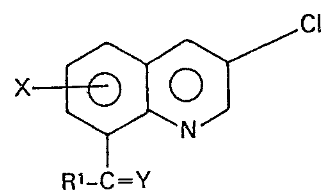

I,

in der

X für Chlor in den Positionen 5, 6 oder 7 steht,

Y für Sauerstoff, Schwefel, Hydroxyimino, zwei Wasserstoffatome, zwei Chloratome oder die Gruppe =N–A–B steht, worin A eine direkte Bindung oder eine $CH_2$-Gruppe und B einen gegebenenfalls durch Chloratome, Nitro-, Methyl- oder Trifluormethyl- oder Methoxygruppen substituierten Phenyl- oder Pyridinrest bedeuten, und

$R^1$ für Wasserstoff, Halogen, Cyano, die Gruppe $-NR^2R^3$ – worin $R^2$ und $R^3$ gleich oder verschieden sind und Wasserstoff, $C_{1-6}$-Alkyl, $C_{1-6}$-Alkenyl, $C_{1-6}$-Hydroxyalkyl, Formyl, Cyclohexyl, Phenyl, Pyridinyl oder beide zusammen den Rest $(CH_2)_4$ oder $(CH_2)_5$, wobei eine $CH_2$-Gruppe durch ein Sauerstoff- oder Stickstoffatom oder eine $N-CH_3$-Gruppe ersetzt sein kann, bedeuten –, eine COOH-Gruppe oder die Gruppe OM steht, worin M ein Metall der 1. und 2. Hauptgruppe des Periodensystems, Wasserstoff, $C_{1-8}$-Alkyl, einen gegebenenfalls durch Halogen, Nitro, $C_{1-4}$-Alkyl oder Trihalogenmethyl substituierten Phenylrest oder den Rest $H_2\overset{\oplus}{N}R^2R^3$, in dem $R^2$ und $R^3$ die oben angegebene Bedeutung haben, darstellt, oder

Y und $R^1$ zusammen mit den C-Atomen eine Nitrilgruppe bedeuten.

2. Verfahren zur Herstellung der Dichlorchinolinderivate der Formel I gemäss Anspruch 1, dadurch gekennzeichnet, dass man eine Verbindung der Formel II

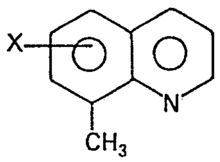

II,

worin X die oben genannte Bedeutung besitzt, mit einem Radikalstarter unter Ausschluss von Licht bei 140–190°C chloriert und die so erhaltenen Verbindungen der Formel III

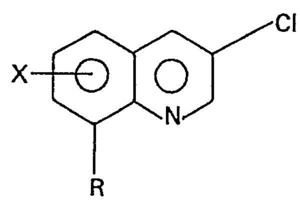

III,

worin X dasselbe wie oben und R Chlormethyl, Dichlormethyl oder Trichlormethyl bedeutet, gegebenenfalls in die Verbindungen der Formel I überführt, in denen der Rest $R^1$–C=Y eine andere Bedeutung als R besitzt.

3. Herbizid, enthaltend eine Verbindung der Formel I gemäss Anspruch 1.

4. Herbizid, enthaltend eine Verbindung der Formel I gemäss Anspruch 1 und einen festen oder flüssigen Trägerstoff.

5. Verfahren zur Herstellung von Herbiziden, dadurch gekennzeichnet, dass man mindestens eine Verbindung der Formel I gemäss Anspruch 1 mit festen oder flüssigen Trägerstoffen mischt.

6. Verfahren zur Bekämpfung unerwünschten Pflanzenwuchses, dadurch gekennzeichnet, dass man die Pflanzen oder den Boden mit einer Verbindung der Formel I gemäss Anspruch 1 behandelt.

## Claims

1. A dichloroquinoline derivative of the formula I

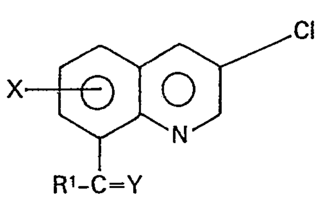

I,

where X is chlorine in the 5-, 6- or 7-position, Y is oxygen, sulfur, hydroxyimino, two hydrogen atoms, two chlorine atoms or =N–A–B, where A is a direct bond or $CH_2$ and B is a phenyl or pyridine radical which is unsubstituted or substituted by chlorine, nitro, methyl, trifluoromethyl or methoxy, and $R^1$ is hydrogen, halogen, cyano or $-NR^2R^3$, where $R^2$ and $R^3$ are identical or different and each is hydrogen, $C_1$–$C_6$-alkyl, $C_1$–$C_6$-alkenyl, $C_1$–$C_6$-hydroxyalkyl, formyl, cyclohexyl, phenyl or pyridinyl, or $R^2$ and $R^3$ together are $(CH_2)_4$ or $(CH_2)_5$, it being possible for one $CH_2$ group to be replaced by oxygen, nitrogen or N–$CH_3$, or $R^1$ is COOH or OM, where M is a metal of main group 1 or 2 of the Periodic Table, hydrogen, $C_1$–$C_8$-alkyl, phenyl which is unsubstituted or substituted by halogen, nitro, $C_1$–$C_4$-alkyl or trihalomethyl, or $H_2\overset{\oplus}{N}R^2R^3$, where $R^2$ and $R^3$ have the above meanings, or Y and $R^1$, together with the carbon atoms, are nitrile.

2. A process for the preparation of a dichloroquinoline derivative of the formula I as claimed in claim 1, wherein a compound of the formula II

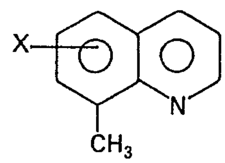

II,

where X has the above meanings, is chlorinated at from 140 to 190°C using a free radical initiator and with exclusion of light, and, if desired, the resulting compound of the formula III

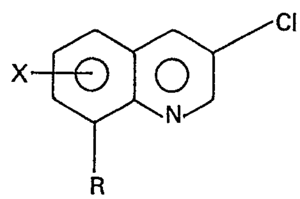

III,

where X has the same meanings as above and R is chloromethyl, dichloromethyl or trichloromethyl, is converted into a compound of the formula I where $R^1$–C=Y is other than R.

3. A herbicide containing a compound of the formula I as claimed in claim 1.

4. A herbicide containing a compound of the formula I as claimed in claim 1 and a solid or liquid carrier.

5. A process for the preparation of herbicides, wherein at least one compound of the formula I as claimed in claim 1 is mixed with a solid or liquid carrier.

6. A process for controlling the growth of unwanted plants wherein the plants or the soil are treated with a compound of the formula I as claimed in claim 1.

## Revendications

1. Dérivés de la dichloro-quinoléine de la formule I

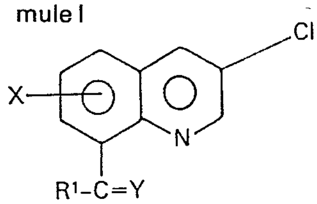

I,

dans laquelle
X désigne un atome de chlore en position 5, 6 ou 7;
Y représente un atome d'oxygène ou de soufre, un groupe hydroxy-imino, deux atomes d'hydrogène ou deux atomes de chlore ou un groupe =N-A-B, dans lequel A représente une liaison directe ou un groupe $-CH_2$ et B un groupe pyridine ou phényle éventuellement substitué par des atomes de chlore ou des groupes nitro, méthyle, trifluorométhyle ou méthoxy, et
$R^1$ désigne un atome d'hydrogène ou d'halogène, un groupe cyano, un groupe $-NR^2R^3$, dans lequel $R^2$ et $R^3$, qui peuvent être identiques ou différents, désignent chacun un atome d'hydrogène, un radical alkyle en $C_1$ à $C_6$, alcényle en $C_1$ à $C_6$ ou hydroxy-alkyle en $C_1$ à $C_6$ ou un groupe formyle, cyclohexyle, phényle ou pyridinyle, ou forment ensemble un groupe $-(CH_2)_4$ ou $-(CH_2)_5$, dont l'un des radicaux $-CH_2$ peut être remplacé par un atome d'oxygène ou un atome d'azote ou un groupe $-N-CH_3$, un groupe -COOH ou un groupe -OM, dans lequel M représente un métal du groupe I ou II du Système périodique des Eléments, un atome d'hydrogène, un radical alkyle en $C_1$ à $C_8$, un groupe phényle éventuellement halo-, nitro-, alkyl (en $C_1$ à $C_4$) - ou trihalométhyl - substitué ou un reste $H_2\overset{\oplus}{N}R^2R^3$, dans lequel $R^2$ et $R^3$ possèdent la signification définie,
Y et $R^1$ pouvant aussi, avec les atomes de carbone adjacents, former un groupe nitrile.

2. Procédé de préparation de dérivés de la dichloro-quinoléine de la formule I selon la revendication 1, caractérisé en ce que l'on soumet un composé de la formule II

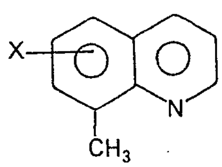

II,

dans laquelle X possède la signification définie plus haut, à une chloration entre 140 et 190°C à l'abri de la lumière et en présence d'un initiateur radicalaire, puis on transforme les composés obtenus, de la formule III

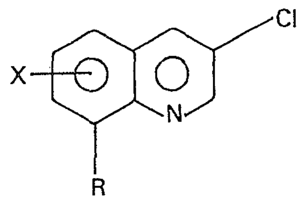

III,

dans laquelle X possède la même signification que ci-dessus et R désigne un groupe chlorométhyle, dichlorométhyle ou trichlorométhyle, le cas échéant en composés de la formule I, pour lesquels le reste $R^1-C=Y$ est différent de R.

3. Composition herbicide, contenant un dérivé de la formule I selon la revendication 1.

4. Composition herbicide, contenant un dérivé de la formule I selon la revendication 1 et une matière support solide ou liquide.

5. Procédé de préparation de compositions herbicides, caractérisé en ce que l'on mélange au moins un composé de la formule I selon la revendication 1 à une matière support solide ou liquide.

6. Procédé de lutte contre la croissance de plantes indésirables, caractérisé en ce que l'on traite les plantes ou le sol avec un composé de la formule I selon la revendication 1.